# EUROPEAN PATENT SPECIFICATION

(11) **EP 1 488 618 B1**
(45) Date of publication and mention of the grant of the patent: **23.11.2011**
(21) Application number: 03704109.2
(22) Date of filing: 11.03.2003
(51) Int. Cl.: H04M 1/247, A61B 5/00

(54) **MULTIFUNCTIONAL MOBILE PHONE FOR MEDICAL DIAGNOSIS AND REHABILITATION**
MEHRFUNKTIONS-MOBILTELEFON FÜR MEDIZINISCHE DIAGNOSEN UND REHABILITATION
TELEPHONE MOBILE MULTIFONCTIONNEL POUR REEDUCATION ET DIAGNOSTIC MEDICAUX

(30) Priority: 12.03.2002 AU PS104802; 05.04.2002 AU PS154702; 22.10.2002 AU 2002952203; 25.11.2002 AU 2002952883
(43) Date of publication of application: 22.12.2004
(73) Proprietor: Era Centre Pty Ltd, Melbourne, VIC 3000 (AU)
(72) Inventor: NOWOSIELSKI, Janusz, Kalorama, VIC 3766 (AU)
(74) Representative: Tonnesen, Bo
(86) International application number: PCT/AU2003/000278
(87) International publication number: WO 2003/077511

(56) References cited:
- EP-A2- 0 987 047
- WO-A-01/70110
- WO-A-99/43136
- WO-A1-00/30529
- WO-A1-01/65810
- WO-A2-02/09396
- DE-A1- 19 947 583
- US-A- 3 974 335
- US-A- 4 964 304
- US-A- 6 100 806
- US-B1- 6 309 117

## Description

### Field of the invention.

The invention relates to the medical tests and rehabilitation treatments provided or controlled by the mobile phone following the programs stored in the memory, loaded from the network, Internet or from the external device.

### Background of the invention.

In recent years the mobile phone became one of the most popular and personal gadgets. Almost every young and older individual use the mobile phone in every day activities.

The system of the mobile phone network very effectively penetrates the society and selectively addresses it's individual members. This advantageous situation creates the opportunity to provide a large number of people with the facilities to conduct the simple medical tests and rehabilitation treatments by extending the mobile phone functional range.

In particular, but not limited to, the screening procedures for early detection of hearing and vision impairments could benefit from this invention. More over the effectiveness of such screening procedures will significantly increase, because the number of tests and time of their performance do not depend upon the often limited time schedule and number of the specialized medical centers. According to the invention the user of the mobile phone can perform the basic medical tests and rehabilitation treatments for himself and family members at convenient time, many times at home without paying the costly visits to the medical centers.

According to the invention the functional extension of the mobile phone applications will benefit young babies and children not covered at all or poorly covered by the screening procedures for early detection of hearing impairments.

Late detection will also significantly delay the child development and affect it's life in future.

The effectiveness of the current screening methods to detect early the hearing impairments in babies and young children is poor or nonexistent in some countries.

Early detection of hearing impairments in very young children still represents a great challenge for the clinicians despite significant advances in technology, particularly digital electronics and computing.

This results from the long existing stigma in clinical methodology limiting the conduct of the testing to specialized centers and well qualified specialists needed to carry out such testing. This imposes a high costs on testing and poor screening efficiency limiting the tests application to only a narrow group of the population. Therefore, not every baby has a chance to be tested at the right time and usually it is brought to the specialized audiological center by parents warring about their baby poor reaction to the environmental sounds and their voices. This visit occurs usually too late, when a baby already passed a critical time for the brain centers development related to the sound stimulation.

The integrity of a sensory system depends on external stimulation. Deprivation studies conclusively showed that sensory stimulus is needed for the structural development of sensory neurons as well as their functional connectivity (Webster & Webster, 1997).

Studies on children's development showed that appropriate sensory stimulation, especially between the ages of 0 to 2 years, is critical for proper development in later years (Ross, Backet & Maxon, 1991)

If baby turns it's head towards the sound source it means not only that it can hear, but also that a proper connectivity between the hearing and motor neurons was established during the critical time. The current method for hearing screening of babies only a few months old, has the following shortcomings:
a/ the baby has to be transported to the specialized hearing center, one or two parents have to dedicate a special time and means for this visit,
b/ in a new strange environment, the baby seldom cooperate with the tester, testing takes too long or it cannot be completed during the first visit,
c/ testing requires the highly specialized, expensive instrumentation and the specialists involvement to carry on the test, and centers patient efficiency is poor,
d/ these centers cannot provide services for mass screening programs and typically test only those babies, who have already been noted by parents or family doctor as having the hearing problems and usually this happen too late.

The Australian Consensus Statement on Neonatal Hearing Screening, Adelaide, March, 2001 states that: " The average age of diagnosis of hearing impairment in centers which screen only infants known to have pertinent risk factors is estimated at 24 months.

Data from Australian Hearing indicate that the median age at detection of children with the most severe hearing impairment (> 90 dB) is between 12 and 18 months while the median age of children with mild hearing loss (< 40 dB) is around 7 years. Effective universal neonatal hearing screening will not replace the need for vigilance and for continued surveillance of hearing behavior and language development to detect hearing impairment in children who have not received neonatal screening or who develop permanent hearing loss at a later age."

It can be noted that planning and implementation of universal neonatal hearing screening is a major effort in many countries across the world being completed in non of them, but only more or less advanced in implementation and still limited to the specialized centers.

There is also a lack of calibrated home device which can be used for hearing test at home for children and adults as a first indicator of hearing problem before more specialized test could be done at the medical center.

According to the invention the mobile phone can also be used for the early detection of vision impairments by the mobile phone users. According to the statistics there are about 45 million blind people worldwide and about 135 million with low vision.

In Australia there are 60,000 blind and 400,000 vision-impaired people.

An early detection of the vision impairments is the best prevention method. Early diagnosis of eye conditions saves vision and even low vision can be useful with adequate vision training and rehabilitation.

Unfortunately there are no regular screening programs and simple tools available for vision testing to every member of the society for early detection of vision impairments.

According to the invention the mobile phone can be modified for mass screening of vision. Today the mobile phone becomes a personal item supporting various needs of increasingly growing number of mobile phone users.

According to the invention it will allow the phone user to use his phone by himself for the purpose of vision screening any time, without costly visits to the specialized centers.

According to the invention the further extension of the mobile functions can benefit the hearing aid and tinnitus maskers users. Current miniaturization of the mobile phones cases and facilities to attach the phone in form of brooch or to place the phone in the upper pocket makes it convenient to extend it's basic functions to perform functions of the hearing aid, tinnitus masker and the remote control for the external hearing aid.

Popular use of the mobile phone with the earphone plugged into the ear is more cosmetically acceptable, than use of the separate hearing aid or tinnitus masker clearly showing the person handicap. More over, the programs stored in the mobile phone memory to run the built in hearing aid, tinnitus masker or remote control for the external hearing aid can be easily modified and updated through the mobile phone network to suit the best the person hearing needs or to optimize the strategy in tinnitus masking treatment.

According a preferred embodiment of the invention the modified mobile phone acoustic channel delivers through the programmable amplifier the amplified environmental noises including speech through the special earphone to the hard of hearing person ear without the necessity to use a separate hearing aid.

Change from the hearing aid mode to the normal mobile phone communication is done automatically by the incoming radio signal or manually by the user, through the mobile phone keypad.

In another extension of the mobile phone functions according to the invention the mobile phone functions provides plurality of special sounds like single tones, white noise, gentle environmental noises for the tinnitus suffers.

These special noises stored in the mobile phone memory or loaded and optionally updated from the network following the clinical progress and strategy in the tinnitus treatment are delivered from the mobile phone acoustic output though the insert earphone to the person ear affected by the tinnitus.

Change from the tinnitus masking mode to the normal mobile phone communication is done automatically when the transmission/receiving mode of operation has been selected or manually by the user, through the mobile phone keypad.

In another extension of the mobile phone functions according to the invention the mobile phone controls parameters of the external heari aid instead of the remote control box used by the hearing aid user and often forgotten to be taken by him when leaving home.

The typical remote control unit provide facilities to turn on/off the hearing aid, to change it's volume and to select the listening program most suitable for given acoustic environment.

Such remote control unit built into the mobile phone duplicates missing remote control box and also according to the invention it can be re-programmed through the mobile phone network to provide the hearing aid user with updated selection of the listening programs most suitable for his needs.

According to the invention the further extension of the mobile phone functions will allow to control built in or externally connected devices typically used for the body or environment temperature measurement, for monitoring the heart beating and respiration rate, for the cardiac and pulmonary auscultation, for the blood sugar level and pressure measurement and also to control devices used in rehabilitation like neuromuscular stimulator.

### Brief description of the Drawings.

- Fig. 1: is a simplified view of the mobile phone with the built in distance measuring sensor and indicator facing the tested ear.
- Fig. 2: is a front view of the mobile phone with the audiogram and and the electronic pointer displayed on the screen.
- Fig. 3: is a simplified view showing the mobile phone acoustic output and supra aural coupler attached to the ear to deliver acoustic test stimuli.
- Fig. 4: is a simplified view showing the mobile phone acoustic output and the insert ear coupler plugged into the ear to deliver acoustic test stimuli.
- Fig. 5: is a simplified view of the mobile phone with the special insert earphone plugged into the ear canal to deliver acoustic test stimuli.
- Fig. 6: is a simplified view of the mobile phone with attached the tube phone delivering acoustic test stimuli to the tested ear.
- Fig. 7: is a simplified view showing the sound level meter to control and calibrate acoustic test stimuli intensities delivered by the mobile phone to the tested ear and placed at the distance set by the telescopic rod.
- Fig. 8: is a simplified view showing the personal sound level indicat attached to the ear with the optional external indicator.
- Fig. 9: is a simplified view of the mobile phone with the attached sound level meter and the distance measuring telescopic rod with built in the microphone.
- Fig. 10: is a view of the attached or built into the mobile phone a distance evaluating device based on a few narrow light beams crossing at the point close to the tested ear.
- Fig. 11: is a view of the mobile phone with built in or attached indicator to set up the correct distance between the tested eye and the mobile phone screen.
- Fig. 12: is an example of the correlation between the vision test patterns and the associated alphanumeric signs to facilitate the use of the mobile phone keypad for the vision test.
- Fig. 13: is an example of the vision test pattern displayed on the mobile phone screen for the color vision test.
- Fig. 14: is a simplified block diagram of the mobile phone with the acoustic channel modified to perform additionally the hearing aid function.
- Fig. 15: is a simplified block diagram of the mobile phone with acoustic channel modified to perform additionally the tinnitus masker function.
- Fig. 16: is a simplified block diagram of the mobile phone with the built in remote control of the hearing aid.
- Fig. 17: is a simplified block diagram of the mobile phone with the optionally connected external devices for the medical tests or rehabilitation controlled by the programs stored in the mobile phone memory.

The invention will be further explained in the detailed description of the preferred embodiments and methods.

In a typical mobile phone the different ring signals can be pre-selected from the memory bank and their volume is set to the predetermined level by the operator using the keypad built into the mobile phone.

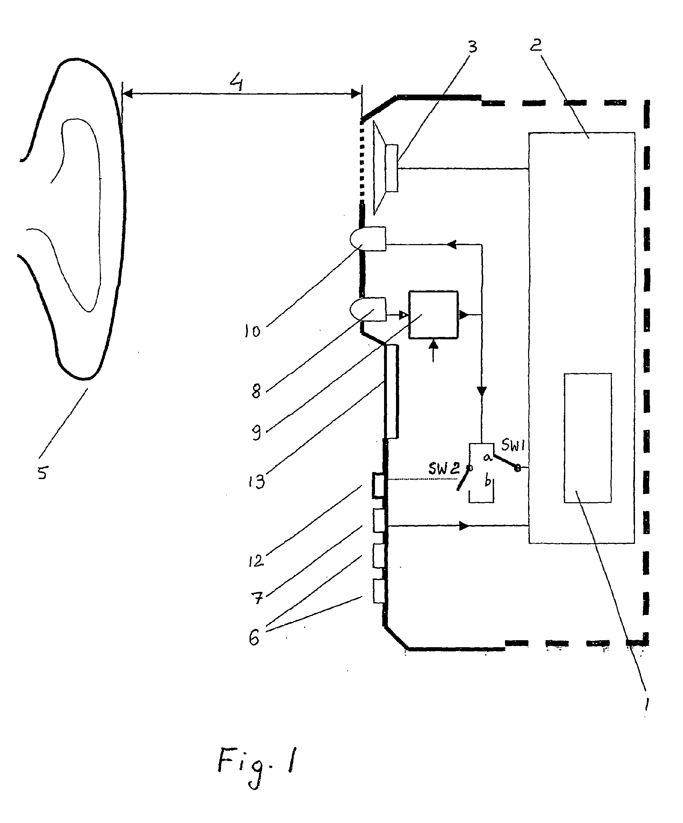

According to the invention presented in Fig.1, the complex sounds limited to the narrow frequency band or single frequency tones are stored in a digital form in the memory bank 1 of the mobile phone electronic circuit 2 in the same way as the ring signals in the ordinary mobile phone.

The intensity level of these sounds presented at the output speaker 3 of the mobile phone is set by the volume control already incorporated in the phone, but for the purpose to conduct the hearing test these intensities are calibrated in dB scale of the Hearing Threshold Level (HTL) for the specified distance 4 between the mobile phone speaker 3 and the patient's ear 5.

Three modes of operation are possible:
a/ activating the sound "on" manually at the specified distance 4 only approximately estimated by the tester,
b/ activating the sound "on" automatically only at the specified distance 4 when the mobile phone is placed in the correct position,
c/ activating the sound "on" manually at the desired time, but only when the mobile phone is placed at the specified distance 4.

In the first mode of operation the tester positions the mobile phone at the distance he estimates as being close or equal to the specified distance 4 and presses button 7 to activate "on" the testing sound stored in the mobile phone memory 1 selected by him prior to the test.

In the second mode of operation the distance measuring sensor 8 sends the control signal to the one input of the comparator 9 whose second input receives the reference signal Vref. When the mobile phone is placed at the specified distance 4 from the patient's ear 5 then both input signals of the comparator 9 should be the same and then the output signal from the comparator 9 through the changeover switch SW1 set in position "a" is delivered to the mobile phone complex circuitry 2 to activate "on" the testing sound. Additionally the light indicator 10 controlled also from the output of the comparator 9 confirms correct position of the mobile phone. In this mode of operation both sound and light indicator are turned "on" automatically, when the distance of the mobile phone speaker 3 from the patient's ear 5 is the same as the specified distance 4.

In the third mode of operation switch SW1 is set in position "b". When the mobile phone is positioned at the specified distance 4, then only light indicator 10 will come "on" indicating to the operator correct distance between the patient's ear 5 and the speaker 3. The testing sound can be delivered to the patient later by pressing at the desired time button 12 which will close the switch SW2, providing that the position of the mobile phone did not change ea. the light indicator 10 stays "on".

In the second and third mode of operation the action of the distance measuring sensor 8 ensures, that the intensity of the testing sound delivered to the patient's ear 5 will meet the calibration requirements at the specified distance 4.

To test the hearing by the patient himself, he has to select using the mobile phone keypad 6 the type of the acoustic stimulus, it's intensity level he wants to hear from the specified distance 4 and mode of operation.

While holding the mobile phone at this distance from his ear 5 he presses the "on" switch 7 to activate the sound "on" in the first mode of operation.

For given frequency band or single tone, the tester can change the stimulus intensity level using the keypad 6 to find out, what is the lowest level of the acoustic stimulus he can hear. After that, he marks this level on the external audiogram form or using an electronic pointer controlled by the keypad 6, he marks this point on the audiogram showed in the display window 13.

If the second mode of operation has been chosen by the patient, then he has to move the mobile phone at the ear level out of the ear and at the specified distance 4 the testing sound will be automatically produced.

In the third mode of operation the patient position the mobile phone at the specified distance 4 and when the light indicator 10 is "on", he presses the button 12 to close "on" the switch SW2.

The hearing test can be performed by the operator himself or in case of disabled person, young children or babies the second person can perform the test in any of these 3 modes of operation.

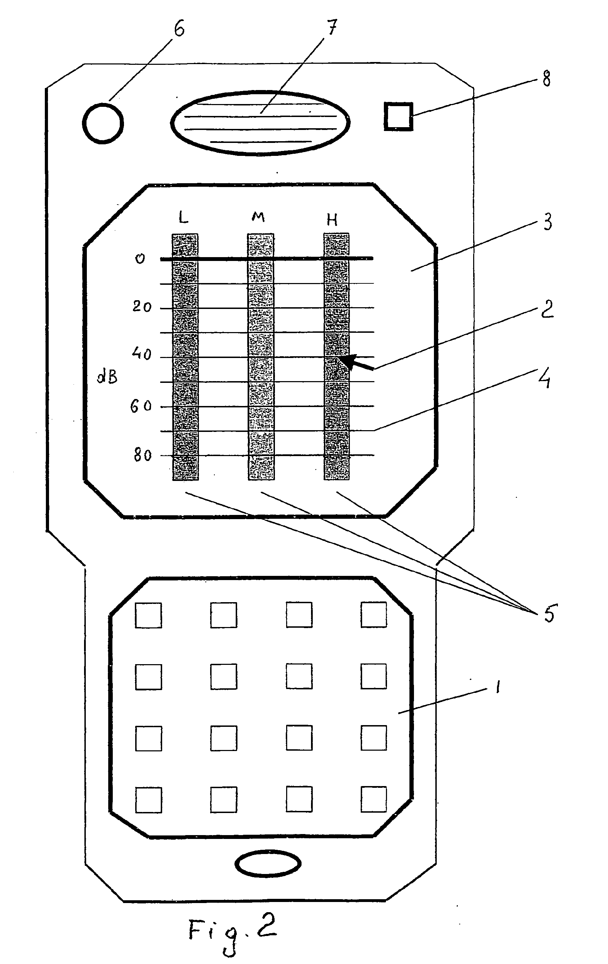

Setting the test parameters such as stimulus frequency and intensity and also recording of the test results can be done in a simple way showed in Fig. 2.

In this drawing the testing point is chose, using the keypad 1 and the electronic pointer 2 directly on the audiogram presented on the display 3 of the mobile phone.

Using the electronic pointer 2 and the keypad 1 desired frequency and intensity of the acoustic stimulus can be simultaneously selected for the given test point in an easy way directly on the displayed audiogram prior to the test.

After the stimulus presentation at the specified distance, the ç tester will decide whether to change the stimulus frequency and/or intensity or accept them depending whether the stimulus was heard or not. If the tester accepted the stimulus as being heard then he will press the enter button or other nominated key of the keypad 1 to store the result of the hearing test in the mobile phone memory and to display it on the audiogram.

Repeating this procedure for all frequency bands or single tones will allow to build an audiogram for each ear.

As an example the mobile phone display 3 shows a few frequency bands 5: low L, medium M and high H in vertical plane and the horizontal lines 4 indicate the stimulus intensity level in dB of the Hearing Threshold Level for the specified distance between the patient's ear and the mobile phone speaker 7.

The distance measuring sensor 6 is placed at the speaker 7 side.

The light indicator 8 normally also placed at the speaker side, in case of testing a small children can be turned "off" and another light indicator placed at the back of the mobile phone can be used instead, to avoid the child false response to the light instead to the testing sound.

Using the electronic pointer 2 the tester can also mark the results of the hearing test directly on the displayed audiogram and store the final results of the test in the mobile phone memory. The data of the stored hearing test results can be sent by the mobile phone to the medical center or to the telemedicine network for the further patient management.

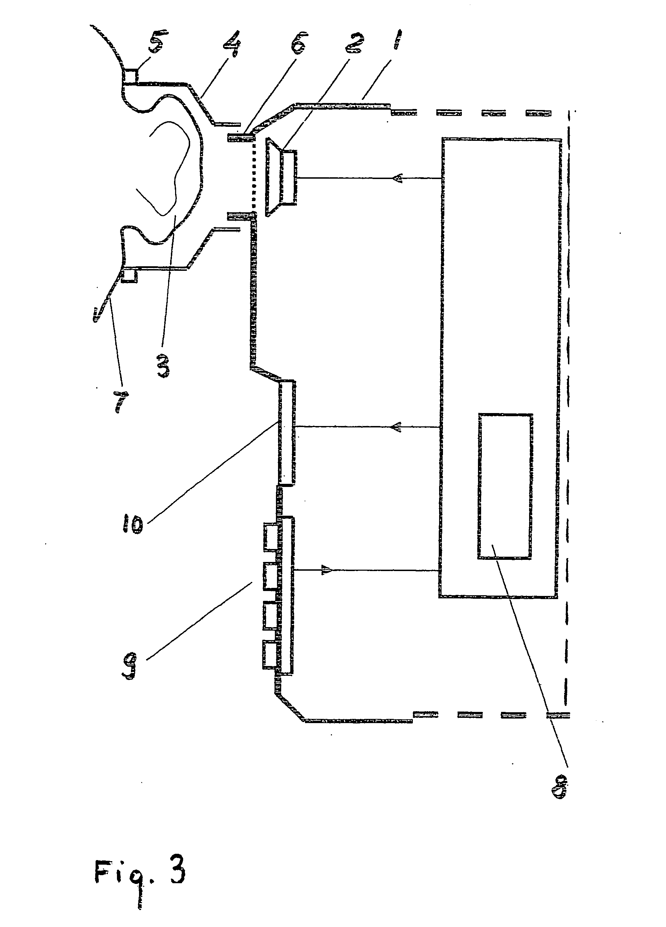

In another embodiment of the invention presented in Fig. 3, the mobile phone 1 adopted for the purpose to perform the hearing test has the output speaker 2 coupled with the ear 3 by means of inserting the coupler 4 with the seal 5 between the mobile phone acoustic output 6 and the patient head 7.

The acoustic sounds patterns for the purpose of the hearing test can be stored in the mobile phone 1 internal memory 8 in a similar way like the set of the phone ring tones is stored, or they can be loaded from the external source into the mobile phone 1 internal memory 8 following the practice allowing to customize the ring tone. Selection of testing sounds patterns and their intensities calibrated in the dB scale in reference of the Hearing Threshold Level is controlled from the mobile phone 1 keypad 9 and showed on the display 10. After the testing sound pattern and it's intensity are selected, then the sound presence is activated by one of the keypad 9 button and the test result can be stored in the mobile phone internal memory for further data processing.

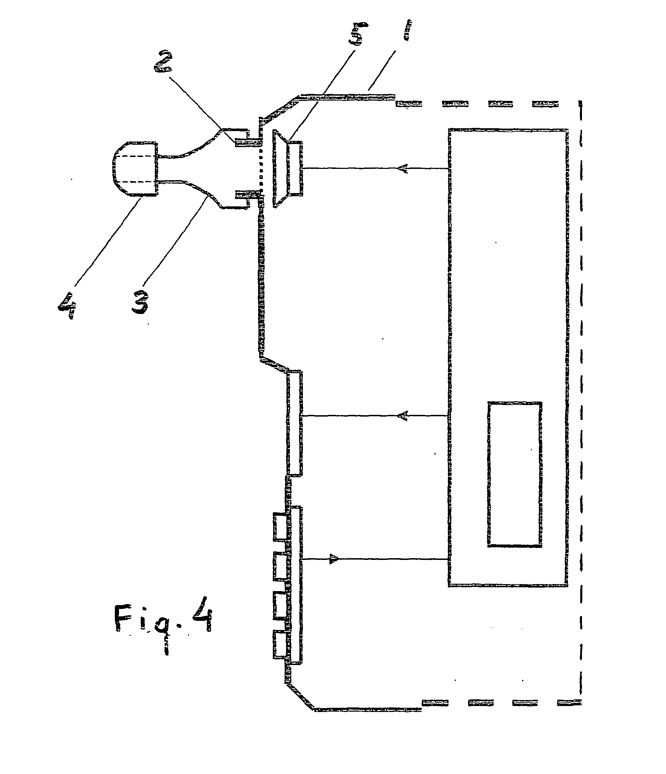

In another embodiment of the invention presented in Fig. 4 the mobile phone 1, has attached to the mobile phone body 2 a speculum 3 with disposable ear tip 4, which is inserted into the patient ear and allows the direct acoustic coupling with the ear canal for the purpose of conducting the hearing test. The testing sound from the speaker 5 is delivered to the patient ear through the speculum 3 and the ear tip 4. The ear tip 4 can be changed according to the patient ear canal size and the whole attachment formed by the speculum 3 and the ear tip 4 can be removed when the mobile phone normal operation is required.

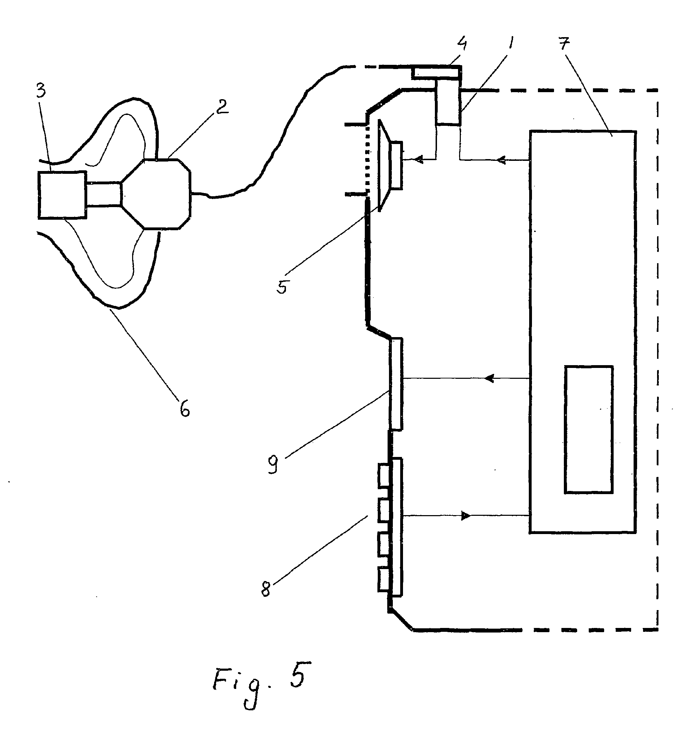

In another another embodiment of the invention presented in Fig. 5 the mobile phone is provided with the socket 1 allowing to plug in preferably, but not necessary, the audiometric insert earphone 2 with the ear tip 3 and plug 4, which when plugged into the socket 1 disconnects the speaker 5 and takes over the output signal from the mobile phone and delivers the testing sound to the patient's ear 6. When the mobile phone is provided by the producer together with the insert earphone for the hearing testing , then the testing sounds intensity produced by this earphone is calibrated in reference to the Hearing Threshold Level by the mobile phone internal calibrator and such intensity level is selected by the keypad 8 and showed by the display 9.

When the insert earphone 2 is purchased separately from the mobile phone, then it is provided with the reference table or with the bar code which contains the data of voltages required for each frequency band of the complex testing sounds or single tones to calibrate the mobile phone for each sound in reference to the Hearing Threshold Level for the individual insert earphone characteristic.

Such voltages, expressed either in analog values or in a special code, are transferred into the mobile phone digital circuitry 7 using the mobile phone keypad 8 or using the mobile phone digital input connected to the external bar reading device which allows to use the bar code automatic data transfer.

Alternatively the mobile phone calibration is done automatically or updated with the data of the earphone characteristic provided by the mobile phone network bank, storing these data for the individual insert earphones recognized by their code numbers.

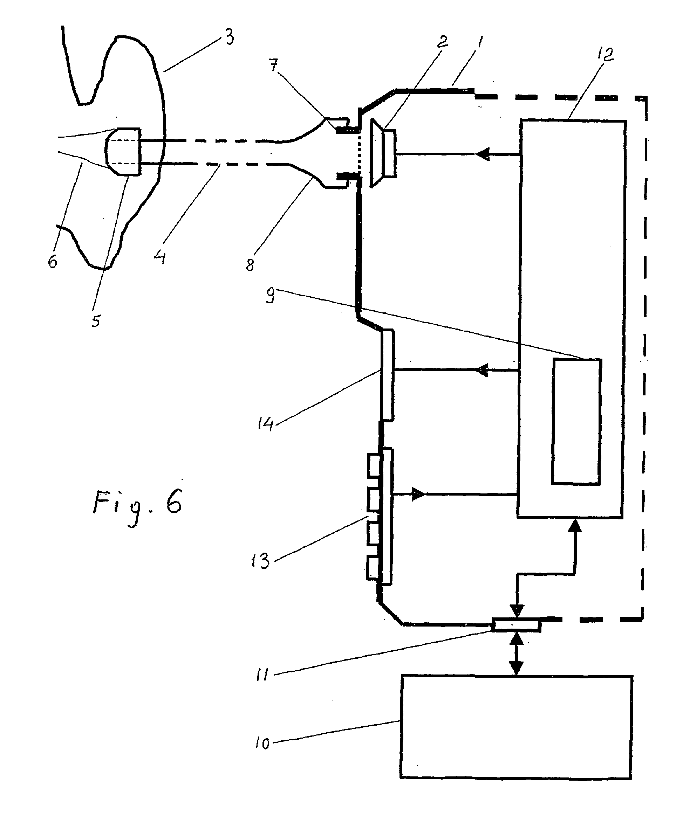

In another embodiment of the invention, the Fig. 6 is a block diagram of the mobile phone 1 adopted for the purpose to perform the hearing test, whose output speaker 2 is coupled with the ear 3 by means of the phone tube 4 and tip 5 plugged into the ear canal 6. The phone tube 4 is attached to the mobile phone acoustic output 7 by the coupler 8.

The acoustic sounds patterns for the purpose of the hearing test can be stored in the mobile phone 1 internal memory 9 in a similar way like the set of the phone ring tones is stored or they can be loaded from the external module 10 connected to the socket 11 which allows the module 10 to communicate with the mobile phone electronic circuitry 12 and, but not necessary with the memory 9.

Selection of testing sounds patterns and their intensities calibrated in the dB scale in reference to the Hearing Threshold Level is controlled from the mobile phone 1 keypad and showed on the display 14. After the testing sound pattern and it's intensity are selected, then the sound presence is activated by one of the keypad 13 button and the test result can be stored in the mobile phone internal memory for further data processing.

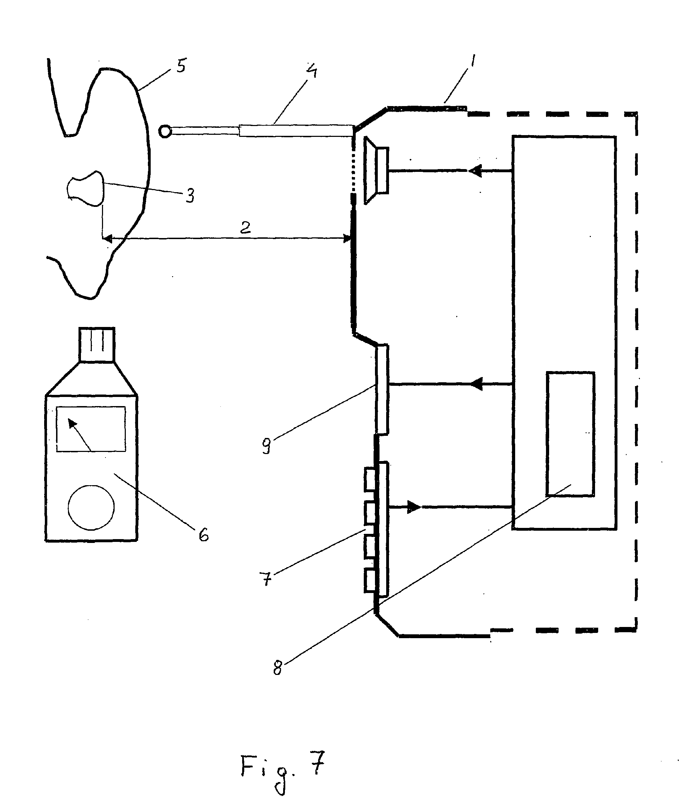

In another embodiment of the invention the Fig. 7 is a block diagram of the mobile phone 1 placed at the distance from the ear entrance 3 controlled by the rod 4 attached to the mobile phone 1 but not touching the ear auricle 5.

The sound level meter 6 measures the sound intensity level close to the ear entrance 3 delivered from the mobile phone 1.

Selecting with the keypad 7 type and volume of each testing sound, one can calibrate in dB the sound intensity approaching the ear entrance 3 and store these levels in the mobile phone 1 memory 8. Then the type of the testing sound and it's calibrated intensity can be read from the display 9.

After the mobile phone calibrated as described above one can perform the hearing test presenting the calibrated testing sounds to the ear at the constant distance 2 controlled by the rod 4.

Instead of the commercial sound level meter 6 the special personal sound level indicator described in the Fig. 2 can be used to calibra the intensity of the testing sounds.

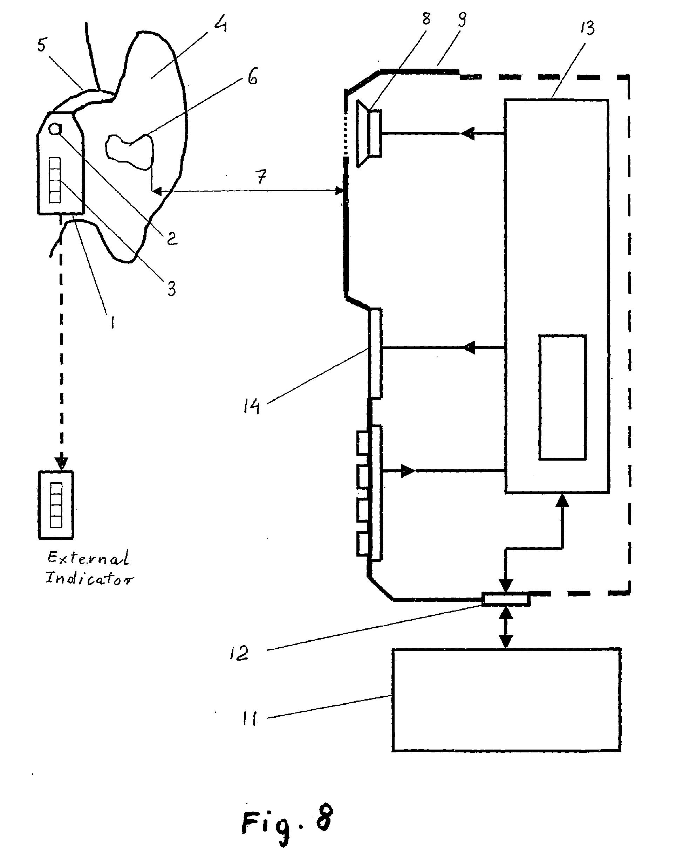

In another embodiment presented in Fig. 8 the personal sound level indicator 1 has a built in the microphone 2 and one or more light indicators 3, to indicate one or more sound intensity levels like 20 dB, 30 dB, 40 dB etc.

One of the light indicator different in color, shape or placement position to the other indicators is used to indicate, that the sound received by the microphone 2 has intensity exceeding the maximum level of the personal indicator scale or predetermined single level typically used in the screening procedures.

The personal sound indicator 1 can be attached to the ear 4 by means of the special hook 5 or a typical head band. It also can be clipped to the ear 4 or hairs like a small brooch or it can be plugged into the ear canal 6 providing a free entrance for the sound through the special opening.

In self hearing test a person with the sound indicator 1 placed closed to the ear canal 6, can observe in the mirror placed in front of it' face, which indicator is "on" during the sound presentation, to assess the level of the sound approaching the ear canal 6.

Instead of the mirror an external light indicator 4 or a special meter can be plugged into the personal sound indicator 1 and conveniently placed in front of the person.

Alternatively, the personal sound indicator 1 with it's light indicator 3 can be placed in front of the person and only the microphone 2 is placed near or plugged into the the ear canal 6. Desired level of the testing sound can be set up by varying the distance 7 between the ear canal 6 and the mobile phone speaker 8 and/or by adjusting the mobile phone 1 volume control.

By changing the distance 7 while presenting at the same time the testing sound from the mobile phone and observing the light indicator 1, one can find out at which sound intensity level he can or cannot hear the testing sound.

The mobile phone 9 has stored in it's memory 10, among the ring tones, the plurality of the special hearing test signals in form of the single tones or complex sounds like the environmental filtered sounds of animals, birds etc.

The hearing test signals could be loaded into the mobile phone memory 10 through the radio link, from the Internet or from the external device 11 plugged into the socket 12, which is connected to the mobile phone 9 electronic circuitry 13. Loading of the testing sounds data from the external device 11, through the radio, infrared or ultrasound link will not change the principle of the invention.

The testing sounds could be also composed by the mobile phone user the same way like he can compose his own ring tones using the mobile phone keypad. In this case, an additional instruction, printed or displayed on the mobile phone display 14 or available from the Internet will be provided to the mobile phone user, to help him to compose the testing signals, especially for the complex sounds, if the sound spectrum has to be more accurately defined. The personal sound indicator and the mobile phone can also be used for the hearing screening test particularly in babies and young children. In this case the second person, holding the mobile phone, approaches the baby from the side position, observes the light indicators and reaction of the baby to the testing sound.

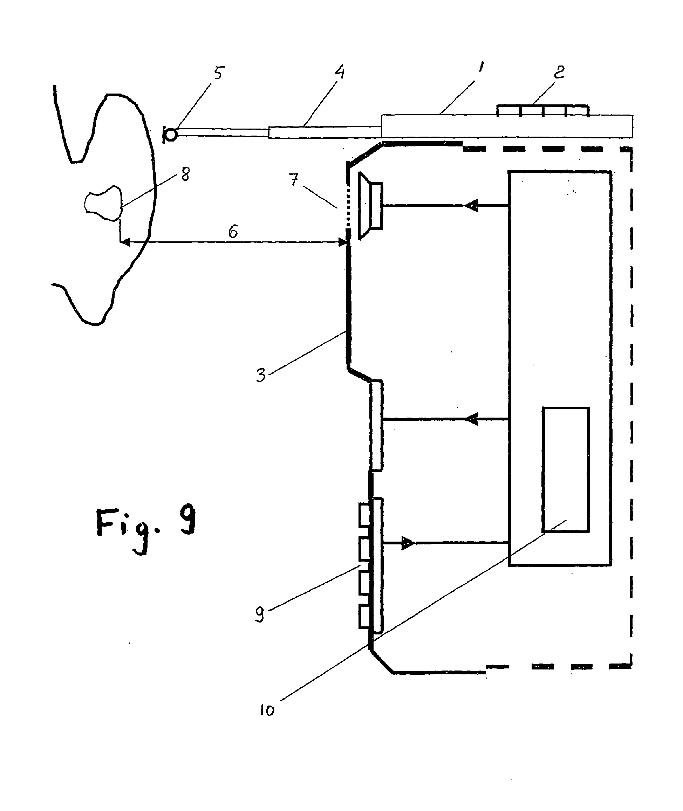

In another embodiment presented in Fig. 9 the personal sound level indicator 1 with it's light indicator 2 is attached to the mobile phone 3 and has a distance rod 4 with the microphone 5 at the end of this rod 4 connected to the personal sound level indicator 1. The constant distance 6 between the speaker output 7 and the ear canal 8 is controlled by the length of the rod 4. Using the keypad 7 one can choose the type of the testing sound stored in the memory 10 and through the volume control adjust the sound intensity level following the indication of the light indicator 2.

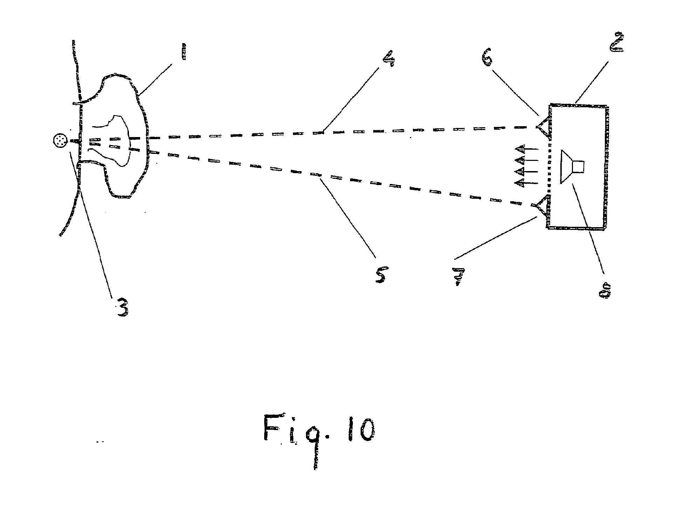

In another embodiment of the invention presented in Fig. 10 the specified distance between the ear 1 and the mobile phone 2 can be estimated by creating near the entrance of the ear 1 the visible light dot 3 formed by the crossing point of two narrow light beams 4 and 5 send by the light sources 6 and 7 such as microlaser diodes or other focused light emitters. At the specified distance only one dot 3 will be visible indicating a proper distance between the ear 1 and the sound module or mobile phone 2. Otherwise two light dots will be visible. The tester moves the sound module or the mobile phone 2 towards the ear 1 to obtain only one dot and then the testing sound can be turned "on" by the tester and delivered from the speaker 8 to the ear 1 to test the hearing.

The mobile phone user will also be able to use his phone for the purpose of vision screening. There is a number of vision tests which can be incorporated into the mobile phone system and it's software program. Two examples of the basic tests for the contrast sensitivity and color discrimination will be presented, but it will not limit plurality of tests possible to perform using the mobile phone. To conduct the vision tests, first it is important to determine the distance between the mobile phone screen and the person eyes.

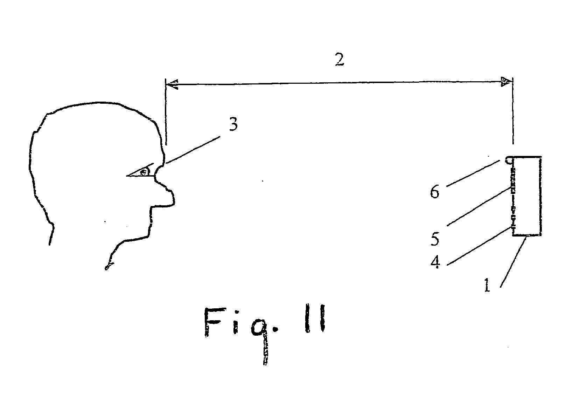

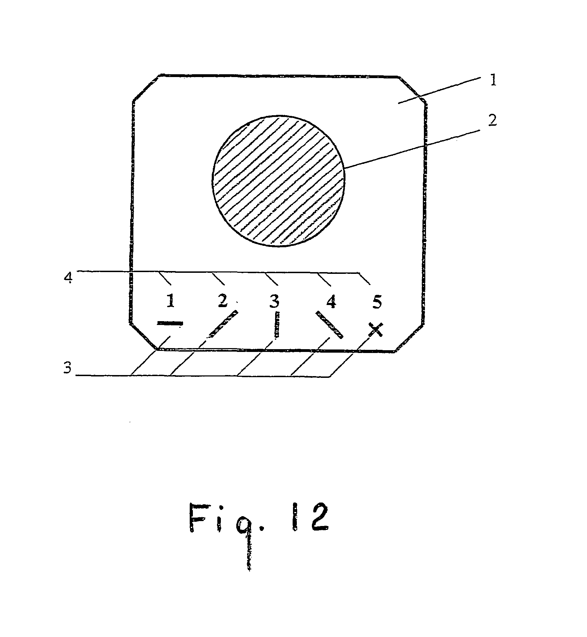

This is presented in Fig. 11. In this figure the mobile phone 1 is held by the person performing the vision test at the specified distance 2 which for some of the tests may correspond to the full length of the person arm. The tested person measures with the separate tape or the pulled out of the phone body mini tape, telescopic or one piece rode the distance between the mobile phone screen 5 and the person eyes 3 and keys value of this distance through the mobile phone keypad 4 into the mobile phone uprocessor controlled electronic system. This allows, following the software program, to automatically adjust the size of the pictures displayed on the screen 5 for the purpose of the vision test. Alternatively, the distance measuring sensor 6 can automatically adjust the size of the images displayed on the screen 5 sending to the mobile phone electronic circuitry which controls the image size, the software data related to the distance. The typical image displayed on the mobile phone screen for the contrast sensitivity test is presented in Fig.12. In this test the image presented on the mobile phone screen 1 as the circle 2 consists a number of parallel lines, which can be positioned vertically, horizontally or at + or - 45 degrees. The tested person has to correlate position of the lines within the circle 2 with one of the symbols 3 displayed on the same screen 1 and has to key in through the mobile phone keypad, one number from the numbers set 4 displayed on the same screen 1 and positioned against the symbols 3. Position and/or density of lines in the circle 1 and also the size of the circle 2 are automatically changed due to the software program for the vision test stored in the mobile phone memory every time the number from the set 4 is keyed in. If the tested person with vision impairment is not decided, what is the position of the lines in the circle 2, then the asterix or other symbol of the keypad can be used instead of the numbers from the set 4. Correct, false and undecided scores of the correlation are recorded in the mobile phone memory. The total number of the circles presentation and the final results interpretation included into the operating software depends upon the criteria recommended by the medical specialists for the vision screening method.
The final pass/fail result of this test is displayed on the mobile phone screen 1 for the tested person consideration and recommends the further action like visit to the specialized medical center.

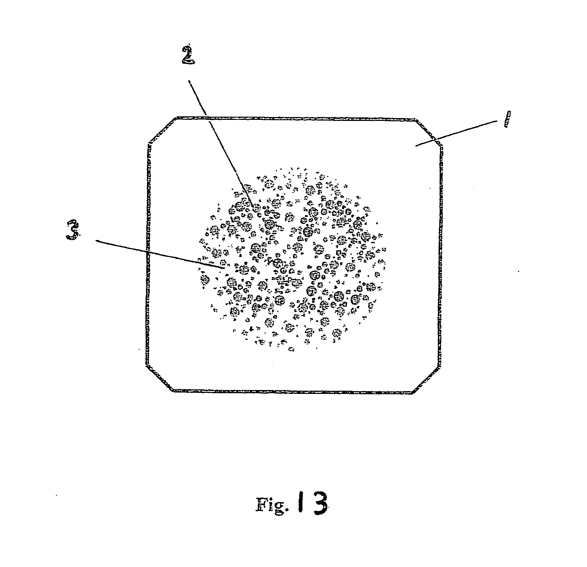

In the second example for the color vision test presented in Fig. 13 the mobile phone screen 3 is used to detect the color vision problems like incorrect color discrimination. In this test the image of number (or letter) 2 built of the same color various diameter and color intensity dots is presented on the background 3 built of multicolor, various sizes and various color intensity dots and displayed on the mobile phone screen 1.
The tested person has to key in through the mobile phone keypad the value of the number (or letter) 1, displayed on the screen 1 against the background 3. The value of the number 2, the colors of it's dots and the background 3 dots, are changed according to the accepted screening method for which the special software is stored in the mobile phone memory.
The total number of the color images presentation and the final results interpretation will depend upon the criteria recommended by the medical specialists for the color vision screening method. The final pass/fail result and eventually more detailed comments are displayed on the mobile phone screen 1 or delivered verbally through the mobile phone acoustic output for the tested person consideration and the further action.

Presented above two examples for the vision tests in no way limit a number of other images and vision tests which could be performed with the mobile phone.
For example, various size numbers and/or letters can be displayed on the mobile phone screen for the tested person to identify them and to key them in through the mobile phone keypad.

Connecting to the mobile phone an external module with the software program to conduct the vision test will not change the principle of this invention in which the vision tests' special images are displayed on the mobile phone screen and the tested person, who looks at these images, has to perform a specific tasks related to these images and has to key in through the mobile phone keypad the results of these tasks for automatic assessment of these results following the test program software and presenting the final results and recommendation for the tested person on the mobile phone screen and/or delivering through the mobile phone acoustic output the verbal instructions stored in the mobile phone memory.
If the larger screen is required, then the mobile phone vision output can be connected to the VDU Monitor or home TV set through a special VDU or RF adapter.

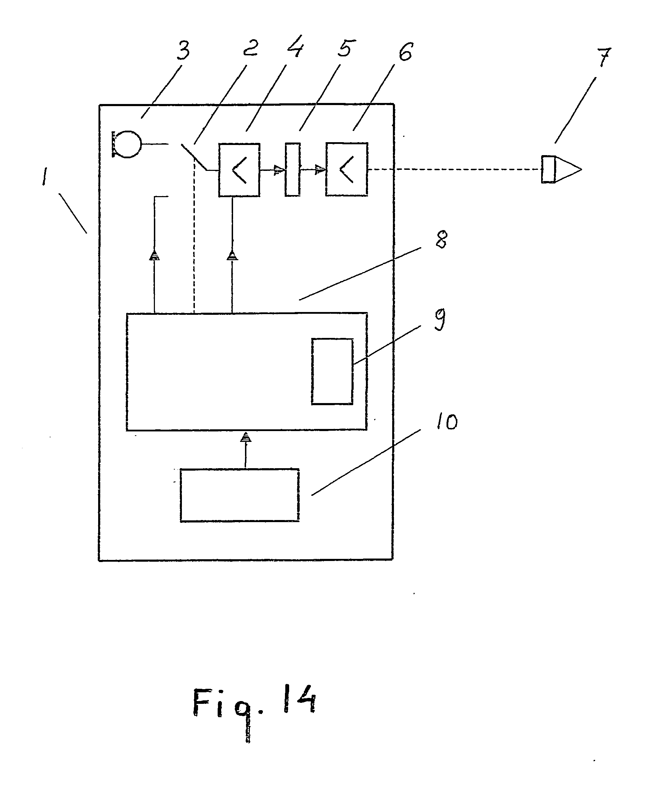

In another embodiment of the invention presented in Fig. 14 the hearing aid forms a part of the mobile phone 1 acoustic channel,in which the changeover switch 2 in it,s normal position takes signal from the microphone 3 and through the programmable amplifier 4, volume control 5 and output amplifier 6 delivers the signal to the external earphone 7 used for the heard of hearing person.
The programmable amplifier 4 adjusts the frequency characteristic and compression of the amplified signal and is controlled by the mobile phone electronic circuit 8 following the data stored in the memory 9. The data stored in the memory 9 could be changed by loading a new data from the mobile phone network or Internet to re-program the hearing aid acoustic parameters.
When the mobile phone is used in normal transmission/receiving mode, then the changeover switch 2 is switched by the electronic circuit 8 to it's second position to receive acoustic signal coming from the network. In this mode of operation the signal coming from the network is processed by the hearing aid channel comprising the programmable amplifier 4, volume control 5, output amplifier 6 and the external earphone 7. The changeover switch is automatically switched by the electronic circuitry 8 when the mobile phone is set to the transmission/reception mode or manually through the mobile phone keypad 10, when the user decides to turn off the hearing aid.

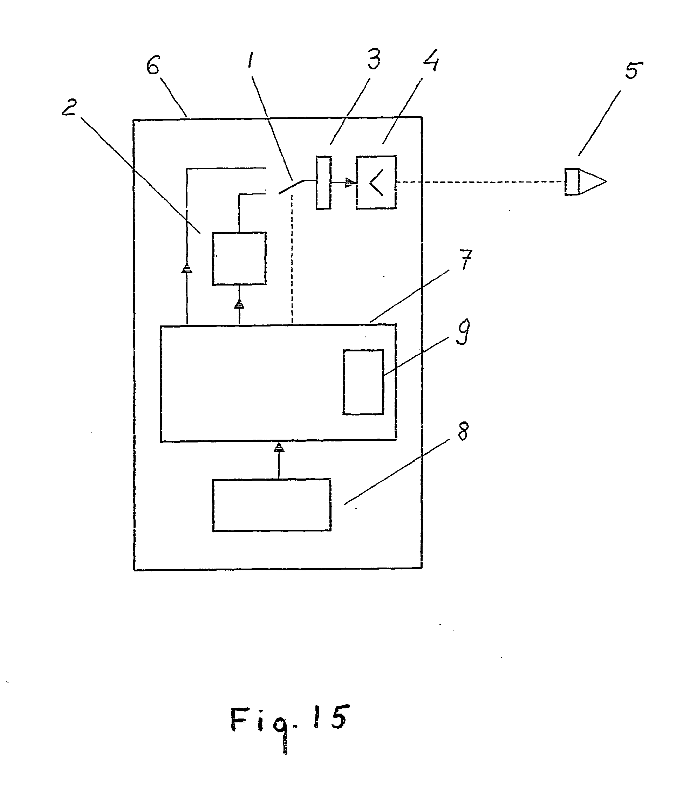

In another embodiment of the invention presented in the Fig. 15 the changeover switch 1 shown in it's normal position processes the special signal like white noise, tones or environmental sounds stored in the memory 2 for the purpose of the tinnitus masking. Next the signal from the switch 1 is delivered through the volume control 3 and the output amplifier 4 to the external earphone 5 used by the tinnitus sufferer. When the mobile phone is set to it's transmission/receiving operation mode, then mobile phone 6 internal electronic circuitry 7 automatically changes position of the switch 1 to carry on this mode of operation.
Changing the mobile phone function from the transmission/receiving mode to the tinnitus masker mode can also be done manually through the mobile phone keypad 8.
The data of the tinnitus masking signals stored in the memory 2 can be changed by loading from the mobile phone network or Internet another data for the new signals.

Additionally the date of the treatment program like changing periodically type of masking signals are stored in the memory 9 and can be updated through the mobile phone network or the Internet.

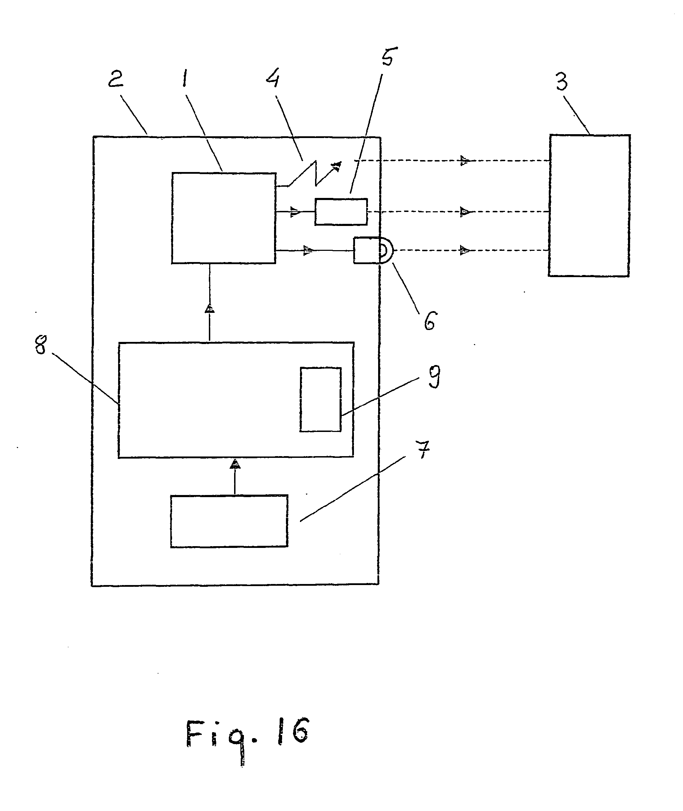

In another embodiment of the invention presented in the Fig. 16 the remote control unit 1 typically used to select functions and the listening programs of the hearing aid is built into the mobile phone 2. The control unit 1 can communicate with the remote hearing aid through the radio wave output 4, magnetic or ultrasound transducer 5 or or infrared transducer 6.
Selection of the hearing aid functions or listening programs is done through the mobile phone keypad 7 connected to the mobile phone electronic circuitry 8. The memory 9 stores a number of commends allowing to select through the remote unit 1 and the electronic circuitry 8 the desired listening programs and the hearing aid functions like microphone, telecoil, volume level and on/off mode of operation. The number of these commends can be changed or optionally updated through the mobile phone network or the Internet.

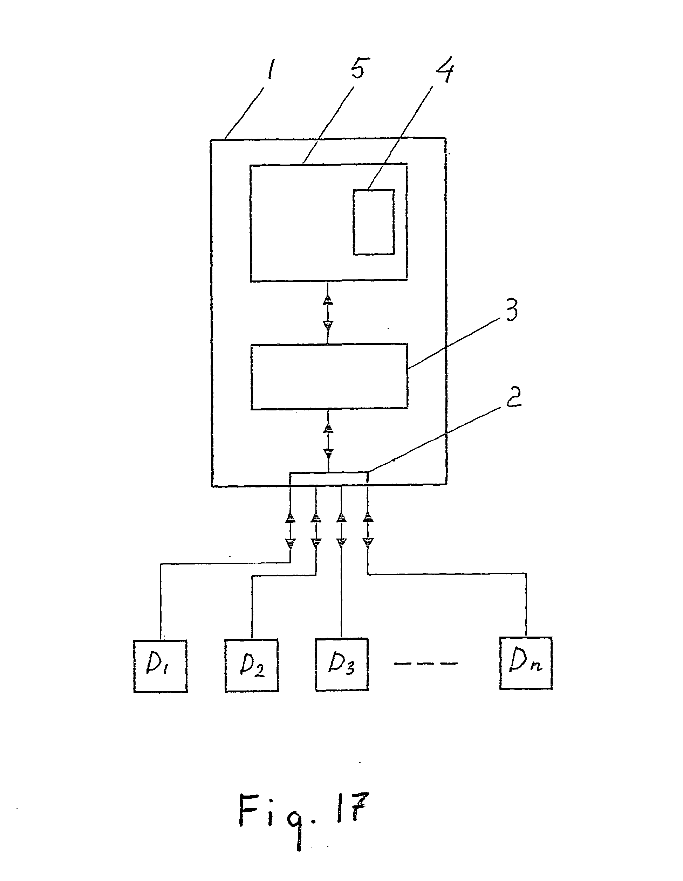

In another embodiment of the invention presented in Fig. 17 a number of the external measuring and monitoring devices D1, D2, D3 ... Dn can be connected individually to the mobile phone 1 external socket to be controlled by the interface 3 and the mobile phone tests or rehabilitation programs stored in the memory 4 being a part of the mobile phone electronic circuitry 5. Alternatively these external devices send the analog or digital data to the mobile phone as the result of their action for further processing.
Various types of the devices measuring the body or environmental temperature, heart beating and lung respiration rate, blood sugar or pressure, monitoring cardiac or pulmonary auscultation, controlling the treatment with the neuromuscular stimulator etc. can be interfaced with the mobile phone through the interface 3 following the standard interfacing rules.

The testing or rehabilitation programs stored in the memory 4 are changed or updated through the mobile phone network or the Internet.

## Claims

1. A mobile phone for performing hearing and vision tests, the mobile phone including:
an acoustic and video channels adapted to provide a definable acoustic output and video display;
the mobile phone allowing communication by mobile telephone network, or internet, or by external device linked to the mobile phone between a source of data tests and the acoustic and video channels;
a mobile phone memory for receiving acoustic and visual test stimuli of a hearing and vision tests from the source, the test stimuli including representations of defined audio output and video display, and wherein the acoustic test stimuli includes representations of a plurality of predefined acoustic outputs which are played back from the mobile phone memory at different frequencies and intensities;
a distance measuring sensor for sensing and determining distance between a person to be tested for a hearing and vision test and the acoustic and video channels;
an operating keypad in which a person to be tested is able to enter test data wherein the mobile phone memory provides instructions to the acoustic output and video channels to emit defined acoustic and video test stimuli.

2. A mobile phone according to claim 1, wherein the distance measuring means includes two spaced apart light sources which are calibrated such that the light sources converge to form a single visible dot at a predetermined distance for conducting a hearing test.

3. A mobile phone according to claim 2 wherein the light sources are selected from micro-laser diodes or focused light emitters.

4. A mobile phone according to claim 1, wherein the test stimuli are able to be updated from a remote source including by the mobile phone including by any one or more of mobile phone network or Internet or an infrared link, ultrasound link or cable from external sources.

5. A mobile phone according to claim 1 wherein the acoustic output is connected to the mobile phone to provide test results to a remote location.

6. A mobile phone according to claim 1, wherein when a correct answer is received by the operating keypad in response to the defined acoustic output and video display of the test stimuli, the acoustic test stimuli frequencies and intensities or video test stimuli is changed.

7. A mobile phone according to claim 1, including an insert earphone interconnected to the mobile phone for maintaining calibrated sound to the ear of the user, wherein the plurality of the acoustic test stimuli is calibrated in frequency and intensity and delivered from the mobile phone directly to the ear by the insert earphone.

8. A mobile phone according to claim 7 wherein the insert earphone includes a special number or bar code providing test stimuli to maintain precise audio calibration together with the receiver of the mobile phone.

9. A mobile phone according to claim 8 wherein the test stimuli is directly entered through the operating keypad of the mobile phone.

10. A mobile phone according to claim 8 wherein the test stimuli is obtained by the special number or bar code and feedback from a remote source by the mobile phone linked to a network to calibrate the mobile phone receiver together with the insert earphone.

11. A mobile phone according to claim 1 when used to control operation of a hearing aid wherein the mobile phone memory includes volume control, output amplifier, external earphone and programmable amplifier controlling the frequency characteristic and compression of the amplified signal following the date of the listening program stored in the mobile phone memory and modified through the mobile phone network or the Internet.

12. A mobile phone according to claim 1 when used to control operation of tinnitus masker for a tinnitus sufferer wherein the mobile phone memory includes volume control, output amplifier, external earphone and programmable amplifier controlling the frequency characteristic and compression of the amplified signal following the date of the listening program stored in the mobile phone memory and modified through the mobile phone network or the Internet to mask the determined tinnitus noise frequencies.

13. A mobile phone according to claim 1 including a device for controlling rehabilitation, the device being connected to an external socket of the mobile phone and being adapted to receive instructions from programs stored in the mobile phone memory circuit via an interface between the memory circuit and the external socket.

## Patentansprüche

1. Mobiltelefon zur Durchführung von Hör- und Sehtests, welches Mobiltelefon folgendes umfasst:
Akustik- und Videokanäle, die für eine bestimmbare Akustik- und Bildausgabe geeignet sind;
wobei das Mobiltelefon Datenkommunikation über das Mobiltelefonnetz oder Internet oder über eine mit dem Mobiltelefon verbundene externe Einrichtung zwischen einer Datentestquelle und den Akustik- und Videokanälen ermöglicht;
einen Mobiltelefondatenspeicher zum Empfang von akustischen und visuellen Teststimuli eines Hör- und Sehtests von der Quelle, wobei die Teststimuli Darstellungen einer bestimmten Akustik- und Bildausgabe umfassen und wobei die akustischen Teststimuli Darstellungen mehrerer vorausbestimmter Akustikausgaben umfassen, welche vom Mobiltelefondatenspeicher mit verschiedenen Frequenzen und Intensitäten wiedergegeben werden;
einen Abstandfühler zum Fühlen und Bestimmen des Abstands zwischen einer Person, die einem Hör- und Sehtest vornimmt, und den Akustik- und Videokanälen;
eine Bedienungstastatur, in welche eine Person, die getestet werden soll, Testdaten eingeben kann, wobei der Mobiltelefondatenspeicher die Akustikausgabe und Videokanäle instruiert, bestimmte Akustik- und Videoteststimuli auszusenden.

2. Mobiltelefon gemäss Anspruch 1, in welchem der Abstandfühler zwei im Abstand voneinander angebrachte Lichtquellen umfasst, die so kalibriert sind, dass die Lichtquellen zur Durchführung eines Hörtests in einem vorausbestimmten Abstand konvergieren, um einen einzelnen sichtbaren Punkt zu bilden.

3. Mobiltelefon gemäss Anspruch 2, in welchem die Lichtquellen unter Mikrolaser-Dioden oder fokusierten Lichtsendern ausgewählt werden.

4. Mobiltelefon gemäss Anspruch 1, in welchem die Teststimuli von einer entfernten Quelle aufdatiert werden können, hierunter über das Mobiltelefon, hierunter über ein beliebiges Mobiltelefonnetz oder das Internet oder einen Infrarotanschluss, Ultraschallanschluss oder ein Kabel von externen Quellen.

5. Mobiltelefon gemäss Anspruch 1, in welchem die Akustikausgabe mit dem Mobiltelefon verbunden ist, um Testergebnisse an eine entfernte Stelle zu senden.

6. Mobiltelefon gemäss Anspruch 1, in welchem die akustischen Teststimulifrequenzen und Intensitäten oder die Videoteststimuli geändert werden, wenn die Bedienungstastatur als Antwort auf die bestimmte Akustik- und Bildausgabe der Teststimuli eine korrekte Antwort erhält.

7. Mobiltelefon gemäss Anspruch 1, welches einen mit dem Mobiltelefon verbundenen Einsteckhörer umfasst, um dem Ohr des Benutzers fortlaufend kalibrierten Schall zuzuführen, in welchem die Frequenz und die Intensität der verschiedenen akustischen Teststimuli kalibriert ist und durch den Einsteckhörer direkt vom Mobiltelefon zum Ohr geliefert wird.

8. Mobiltelefon gemäss Anspruch 7, in welchem der Einsteckhörer eine spezielle Nummer oder einen Streifenkode umfasst zur Aussendung von Teststimuli, um zusammen mit dem Hörer des Mobiltelefons eine präzise Audiokalibrierung aufrechtzuerhalten.

9. Mobiltelefon gemäss Anspruch 8, in welchem die Teststimuli direkt durch die Bedienungstastatur des Mobiltelefons eingegeben werden.

10. Mobiltelefon gemäss Anspruch 8, in welchem die Teststimuli durch die spezielle Nummer oder den Streifenkode erhalten werden und über das Mobiltelefon, welches mit einem Netzwerk verbunden ist, von einer entfernten Quelle wiedergegeben werden, um den Mobiltelefonempfänger zusammen mit dem Einsteckhörer zu kalibrieren.

11. Mobiltelefon gemäss Anspruch 1, wenn es zur Steuerung der Funktion einer Hörhilfe verwendet wird, in welchem der Mobiltelefondatenspeicher Lautstärkeregelung, einen Ausgangsverstärker, externe Kopfhörer und einen programmierbaren Verstärker zur der Frequenzcharakteristik und Kompression des verstärkten Signals zufolge der Daten des im Mobiltelefondatenspeicher gespeicherten Hörprograms und modifiziert durch das Mobiltelefonnetz oder das Internet.

12. Mobiltelefon gemäss Anspruch 1, wenn es zur Steuerung der Funktion eines Tinnitusmaskierers für eine an Tinnitus leidende Person verwendet wird, in welchem der Mobiltelefondatenspeicher Lautstärkeregelung, einen Ausgangsverstärker, externe Kopfhörer und einen programmierbaren Verstärker umfasst zur Steuerung der Frequenzcharakteristik und Kompression des verstärkten Signals zufolge der Daten des im Mobiltelefondatenspeicher gespeicherten Hörprograms und modifiziert durch das Mobiltelefonnetz oder das Internet, um die ermittelten Tinnitusgeräuschfrequenzen zu maskieren.

13. Mobiltelefon gemäss Anspruch 1, welches eine Einrichtung zur Rehabilitierungssteuerung umfasst, welche Einrichtung an einen externen Anschluss des Mobiltelefons angeschlossen und dazu geeignet ist, Instruktionen von im Mobiltelefondatenspeicherkreislauf gespeicherten Programmen über eine Kontaktfläche zwischen dem Datenspeicherkreislauf und dem externen Anschluss zu empfangen.

## Revendications

1. Téléphone mobile pour l'exécution d'examens auditifs et visuels, le téléphone mobile comprenant:
Un canal acoustique et un canal vidéo adaptés pour fournir une sortie acoustique et un affichage vidéo définissables;
le téléphone mobile permettant la communication par réseau de téléphonie mobile, ou par l'internet, ou par un dispositif externe lié au téléphone mobile entre une source d'examens de données et le canal acoustique et le canal vidéo;
une mémoire de téléphone mobile pour la réception des stimuli d'examen acoustiques et visuels d'un examen auditif et visuel à partir de la source, les stimuli d'examen comprenant des représentations d'affichage vidéo et de sortie auditive définis, et où les stimuli d'examen acoustiques comprennent des représentations d'une multitude de sorties acoustiques prédéfinies qui sont lus de la mémoire du téléphone mobile à des fréquences et des intensités différentes;
un détecteur de mesure de distance pour détecter et déterminer la distance entre une personne à être examinée pour un examen auditif et visuel et le canal acoustique et le canal vidéo;
un clavier de commande dans lequel une personne à être examinée est capable d'entrer des données d'examen, où la mémoire du téléphone mobile fournit des instructions à la sortie acoustique et les canaux vidéos d'émettre des stimuli d'examen acoustiques et vidéos définis.

2. Téléphone mobile selon la revendication 1, dans lequel les moyens de mesure de distance comprennent deux sources de lumière espacées l'une de l'autre qui sont calibrées de manière à ce que les sources de lumière convergent pour former un seul point visible à une distance prédéterminée pour conduire un examen auditif.

3. Téléphone mobile selon la revendication 2, dans lequel les sources de lumière sont sélectionnées à partir de diodes micro-lasers ou d'émetteurs de lumière focalisés.

4. Téléphone mobile selon la revendication 1, dans lequel les stimuli d'examen sont capable d'être mises à jour à partir d'une source éloignée, entre autre par l'intermédiaire du téléphone mobile, sous ce titre par un ou plusieurs parmi les suivant moyens, à savoir de réseaux de téléphonie mobile ou de l'Internet ou d'un lien infrarouge, d'un lien ultrason ou d'un câble de sources externes

5. Téléphone mobile selon la revendication 1, dans lequel la sortie acoustique est raccordée au téléphone mobile pour fournir des résultats d'examen à une localisation éloignée.

6. Téléphone mobile selon la revendication 1, dans lequel, lorsqu'une réponse correcte est reçu par le clavier de commande en réponse à la sortie acoustique et l'affichage vidéo définis des stimuli d'examen, les fréquences et les intensités de stimuli d'examen acoustique ou les stimuli d'examen vidéo sont changés.

7. Téléphone mobile selon la revendication 1 comprenant un écouteur interne inter-connecté au téléphone mobile pour maintenir du son calibré à l'oreille de l'utilisateur, où la multitude de stimuli d'examen acoustiques est calibrée en fréquence et en intensité et fournie à partir du téléphone mobile directement à l'oreille par l'écouteur interne.

8. Téléphone mobile selon la revendication 7, dans lequel l'écouteur interne comprend un nombre spécial ou un code à barres qui fournit des stimuli d'examen afin de maintenir la calibration auditive précise avec le récepteur du téléphone mobile.

9. Téléphone mobile selon la revendication 8, dans lequel les stimuli d'examen sont entrés directement par le clavier de commande du téléphone mobile.

10. Téléphone mobile selon la revendication 8, dans lequel les stimuli d'examen sont obtenus par le nombre spécial ou le code à barres et par la rétroaction à partir d'une source éloignée par le téléphone mobile lié à un réseau pour calibrer le récepteur du téléphone mobile avec l'écouteur interne.

11. Téléphone mobile selon la revendication 1 lorsque utilisé pour contrôler l'opération d'un appareil de correction auditive dans lequel la mémoire du téléphone mobile comprend le contrôle de volume, un amplificateur de sortie, un écouteur externe et un amplificateur programmable contrôlant la caractéristique de la fréquence et la compression du signal amplifié suivant la date du programme d'écoute mémorisé dans la mémoire du téléphone mobile et modifié par l'intermédiaire du réseau du téléphonie mobile ou l'Internet.

12. Téléphone mobile selon la revendication 1 lorsque utilisé pour contrôler l'opération de masque d'acouphène pour une personne souffrant de l'acouphène, dans lequel la mémoire du téléphone mobile comprend le contrôle de volume, un amplificateur de sortie, un écouteur externe et un amplificateur programmable contrôlant la caractéristique de la fréquence et la compression du signal amplifié suivant la date du programme d'écoute mémorisé dans la mémoire du téléphone mobile et modifié par l'intermédiaire du réseau du téléphone mobile ou l'Internet pour masquer les fréquences de bruit d'acouphène déterminées.

13. Téléphone mobile selon la revendication 1 comprenant un dispositif pour le contrôle de la réhabilitation, le dispositif étant raccordé à un support externe du téléphone mobile et étant adapté à recevoir des instructions à partir de programmes mémorisés dans le circuit de la mémoire du téléphone mobile par l'intermédiaire d'une interface entre le circuit de mémoire et le support externe.
